(19) Europäisches Patentamt
European Patent Office
Office européen des brevets

(11) EP 3 003 383 B1

(12) EUROPEAN PATENT SPECIFICATION

(45) Date of publication and mention of the grant of the patent:
**03.10.2018 Bulletin 2018/40**

(21) Application number: **14730606.2**

(22) Date of filing: **30.05.2014**

(51) Int Cl.:
*A61K 47/14* (2017.01) *A61K 31/593* (2006.01)
*A61K 31/663* (2006.01) *A61P 19/08* (2006.01)
*A61P 19/10* (2006.01) *A61K 9/20* (2006.01)
*A61K 9/28* (2006.01)

(86) International application number:
**PCT/PT2014/000036**

(87) International publication number:
**WO 2014/193255 (04.12.2014 Gazette 2014/49)**

(54) **SOLID COMPOSITION FOR ORAL ADMINISTRATION CONTAINING IBANDRONIC ACID OR A PHARMACEUTICALLY ACCEPTABLE SALT THEREOF AND VITAMIN D**

FESTE ZUSAMMENSETZUNG ZUR ORALEN VERABREICHUNG ENTHALTEND IBRANDONIC-SÄURE ODER DESSEN SALZE UND VITAMIN D

COMPOSITION SOLIDE POUR L'ADMINISTRATION ORALE COMPRENANT DE L'ACIDE IBANDRONIQUE OU DU SEL PHARMACEUTIQUEMENT ACCEPTABLE ET DU VITAMINE D

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **31.05.2013 PT 10697813**

(43) Date of publication of application:
**13.04.2016 Bulletin 2016/15**

(73) Proprietor: **TECNIMEDE SOCIEDADE TECNICO-MEDICINAL S.A.**
**2710-089 Sintra (PT)**

(72) Inventors:
- **SILVA SERRA, João Pedro**
  **P-2710-089 Sintra (PT)**
- **SOARES, Isabel Maria**
  **P-2710-089 Sintra (PT)**

(74) Representative: **Miranda de Sousa, João Paulo Vaz**
**Garrigues IP, Unipessoal Lda.**
**Av. da República, 25-1°**
**1050-186 Lisboa (PT)**

(56) References cited:
**WO-A1-2008/074144** **WO-A1-2011/063952**
**US-A1- 2005 261 250** **US-A1- 2008 139 514**

- **M Kim: "The safety, tolerability and serum ibandronate pharmacokinetic profile of DP-R206, fixed dose ibandronate and cholecalciferol, combination compared with ibandronate 150mg tablet", , 1 February 2013 (2013-02-01), pages s70-s71, XP055132293, Retrieved from the Internet: URL:http://www.nature.com/clpt/journal/v93/n1s/pdf/clpt2012256a.pdf [retrieved on 2014-07-30]**
- **MILLER T A ET AL: "Pharmaceutical tablet lubrication", INTERNATIONAL JOURNAL OF PHARMACEUTICS, ELSEVIER BV, NL, vol. 41, no. 1-2, 1 January 1988 (1988-01-01), pages 1-19, XP025530943, ISSN: 0378-5173, DOI: 10.1016/0378-5173(88)90130-5 [retrieved on 1988-01-01]**
- **JEON JI-YOUNG ET AL: "Comparison of the Pharmacokinetics, Safety, and Tolerability of Vitamin D3in DP-R206 (150-mg Ibandronate/24,000-IU Vitamin D3Tablet) and as Monotherapy (24,000 IU) in Healthy Male Korean Adults", CLINICAL THERAPEUTICS, EXCERPTA MEDICA, PRINCETON, NJ, US, vol. 36, no. 1, 28 December 2013 (2013-12-28), pages 48-57, XP028670219, ISSN: 0149-2918, DOI: 10.1016/J.CLINTHERA.2013.12.001**
- **"Vegetable Oi, Hydrogenated, Type I" In: Kibbe: "Handbook of Pharmaceutical Excipients", 2000 pages 578-579,**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

(Cont. next page)

• **"Hydrogenated vegetable oil" In: "USP24/ NF19", 1 January 2000 (2000-01-01) pages 2534-2535,**

## Description

### Technical Field

The present invention relates to an improved solid pharmaceutical composition for oral administration comprising ibandronic acid or a pharmaceutically acceptable salt thereof, vitamin D and other adjuvants / excipients which confer desirable physico-chemical properties to a solid formulation for oral administration. The treatment of bone diseases and disorders of calcium metabolism are the therapeutic target of this composition.

### State of the Art

Abnormal bone resorption is associated with various human and other mammal disorders. Such disorders include, amongst others, osteoporosis and Paget's disease. Osteoporosis is the most common disorder of the bone and it is characterized by loss of bone mass and microarquitectural deterioration of bone tissue, leading to a consequent increase in fragility and susceptibility to fracture.

The incidence of this condition has been growing and its occurrence has been associated with increased life expectancy of the human population. The manifestation of the disease is more marked in women during the postmenopausal period.

The administration of bisphosphonates, synthetic compounds characterized by a group P-C-P, analogs of inorganic pyrophosphates are often used in the treatment of bone diseases. Medically they are used to inhibit bone resorption (carried out by multinucleate cells known as osteoclasts).

When administered, the bisphosphonate operate almost exclusively in the bone due to their special affinity for calcium phosphate in its solid form which causes them to bind tightly to mineralized bone. Thus bisphosphonates will operate in the newly formed bone and in the osteoclasts. The effect on osteoclasts is expressed by inhibition of their recruitment, decrease of lifetime (by activating the program of cell death, apoptosis) and inhibition of its biological activity. (Review, Development of biphosphonates, Breast Cancer Res (2002) 4:30-34).

Bisphosphonates are inefficiently absorbed by the gastrointestinal tract (<1%) and this limited absorption is compromised in the presence of food and beverages (except water) which will bind with bisphosphonates reducing its bioavailability. To maximize its bioavailability, it is generally recommended that the patient takes the bisphosphonate with an empty stomach and does not ingest any food within 30 minutes after taking it. Intravenous administration has been used to ensure that the intended dose of bisphosphonate enters the bloodstream. When oral administration is preferred, higher doses are required to achieve the same purpose.

Ibandronic acid is one of the most potent antiresorptive bisphosphonates. This amino bisphosphonate binds to hydroxyapatite in calcified bone turning them resistan to hydrolysis by phosphatases, therefore inhibiting abnormal resorption. This bisphosphonate increases bone mass and decreases the risk of fractures, it is highly effective and well tolerated by women with postmenopausal osteoporosis. Its administration has been used also in the treatment of corticosteroid-induced osteoporosis and in other bone conditions.

Vitamin D can be presented in various forms, the most relevant physiological forms are Vitamin D3 (cholecalciferol) and vitamin D2 (ergocalciferol). Vitamin D has been known for long as a hormone steroid with action in regulating the levels of calcium, phosphorus and in the mineralization and growth of bone.

Vitamin D3 is one of the major forms of vitamin D present in diet and it is the first agent of a cascade of reactions that lead to the active form of vitamin D found in the blood, the 1,25- dihydroxycholecalciferol or 1,25 (OH) D. Vitamin D3 plays a key role in regulating calcium homeostasis . The maintenance of calcium levels is made by a system in which vitamin D3 acts as facilitator of effective absorption of calcium by the intestine, thus improving the distribution thereof and bone metabolism.

In this way, low levels of vitamin D3 are associated to hypocalcemia, secondary hypoparathyroidism, fractures, osteoporosis, amongst other conditions resulting from loss of bone mass. Cholecalciferol is considered to be an unstable substance, being sensitive to oxidation, temperature and humidity, which difficult a stable pharmaceutical formulation.

The state of the art recognizes as effective in the said treatment a combination therapy with bisphosphonates and vitamin D3.

To this regard, some published patent application documents already refer to pharmaceutical compositions of vitamin D and bisphosphonates.

WO2001028564 A1 describes the preparation of a composition containing alendronate and calcitriol (active derivative form of vitamin D3) for the treatment of metabolic bone diseases.

WO2003086415 A1 describes a method for inhibiting bone resorption comprising a pharmaceutical composition of alendronate and vitamin D.

WO2008116809 A1 describes a parenteral pharmaceutical composition comprising a bisphosphonate and vitamin D, and administration of 600.000UI vitamin D once a year.

WO 2008074144 A1 describes compositions containing alendronate and vitamin D and additionally microcrystalline cellulose and an anhydrous form of Sray Dried Lactose. It is said in this document that the stickiness typically associated with bisphosphonic acids, in general, seems to have been overcome simultaneously with the improved flowability when the microcrystalline cellulose (e.g., Avicel 302) and the Lactose Anhydrous (e.g., Spray Dried) are used. Such compositions allow for hardness or dissolution time (DT) levels of the preparation to be sufficient low to reduce or eliminate friability issues, and prevent the composition from crumbling away or not ejecting properly during processing.
M.Kim: "The safety, tolerability and serum ibandronate pharmacokinetic profile of DP-R206, fixed dose ibandronate and cholecalciferol, combination compared with ibandronate 150mg tablet", 1 February 2013 (2013-02-01), pages s70-s71, discloses a solid oral composition comprising a fixed dose combination of ibandronate and cholecalciferol for use in treating osteoporosis. It differs from the subject matter of the present application in the lack of medium chain triglyceride or hydrogenated oil.
Also when formulating together ibandronic acid or a pharmaceutically acceptable salt thereof (ibandronate), with crystalline vitamin D , there are problems related with compression, ejection of the tablets from the compressing machine matrix and consequently problems during the coating process. Simultaneously, flow problems occur while the mixtures are subject to compression.
Despite several efforts and previous attempts to overcome these problems, there still remains the need for further improvement in this technical field, as there still exist the need to provide a pharmaceutical composition comprising both active ingredients with an effective bioavailability of active ingredients, ease of administration, improved dosing compliance and stability of the dosage form.
The present invention is therefore directed to the provision of an improved dosage form of a solid pharmaceutical composition for oral administration comprising ibandronic acid or a pharmaceutically acceptable salt thereof, such as sodium ibandronate, together with vitamin D, which solves the above related problems.

## Brief description of the invention

Thus, the object of the present invention is a solid pharmaceutical composition for oral administration comprising ibandronic acid or a pharmaceutically acceptable salt thereof (ibandronate) and Vitamin D with improved flow, compressibility and ejection properties, which enables ease of administration, improved dosing compliance, consistency, control and bioavailability of active ingredients and an improved stability of the dosage form.
It is also an object of the present invention a process to obtain a solid pharmaceutical composition for oral administration comprising ibandronic acid or a pharmaceutically acceptable salt thereof, such as ibandronate and vitamin D with improved flow, compressibility and ejection properties, which enables ease of administration, improved dosing compliance, consistency, control and bioavailability of active ingredients and an improved stability of the dosage form.
A further object of the present invention is a solid pharmaceutical composition for oral administration comprising ibandronic acid or a pharmaceutically acceptable salt thereof, such as sodium ibandronate, vitamin D together with a suitable amount of medium chain triglycerides or a totally or partially hydrogenated oil to improved flow, compressibility and ejection properties of the composition, thus enabling ease of administration, improved dosing compliance, consistency, control and bioavailability of active ingredients and an improved stability of the dosage form.
Another object of the present invention is a process for obtaining a solid pharmaceutical composition for oral administration with improved flow, compressibility and ejection properties, comprising ibandronic acid or a pharmaceutically acceptable salt thereof, in particular, sodium ibandronate and vitamin D together with a predefined amount of medium chain triglycerides or a totally or partially hydrogenated oil, said method comprising the following steps:

- an initial mixture of ibandronic acid or a pharmaceutically acceptable salt thereof with an appropriate amount of excipients using an appropriate and conventional mixer
- wet granulation of the previous mixture using a granulation equipment (a fluid bed dryer)
- drying of granules
- calibration of the dried granules using an appropriate and conventional sieve
- addition of the excipients presented in the external phase, where the medium chain triglyceride or, a totally or partially hydrogenated oil is included to improve the properties of flow, compressibility and ejection of the composition
- mix the dried granules with the external phase using a proper mixer
- by means of a tablet press machine, compacting the previous mixture into cores or alternatively, using a filling machine for the obtention of capsules or sachets.

Other objects of the invention are defined in the dependent claims.

## Detailed Description of the Invention

The present invention is directed to a solid pharmaceutical composition for oral administration comprising ibandronic acid or a pharmaceutically acceptable salt thereof, such as sodium ibandronate, and Vitamin D, with improved flow, compressibility and ejection properties and which enables ease of administration, improved dosing compliance, consistency, control and bioavailability of active ingredients and an improved stability of the dosage form.

Although the present invention is applicable in general to solid dosage forms for oral administration comprising the said active ingredients, such as capsules, sachets, etc., the claimed advantages of the invention are shown in particular when the solid pharmaceutical composition for oral administration is formulated in the dosage form of a coated or non-coated tablet.

The improved solid pharmaceutical composition for oral administration of the present invention is characterized by having appropriate physical properties which provide the said advantages during use thereof in medicine.

The physical properties shown by the solid pharmaceutical composition for oral administration of the present invention are: improved flow, compression and ejection properties during the manufacture, and as a consequence thereof, adequate hardness and friability and no irregularities on the surface of the tablets.

Appropriate flow is required to manufacture tablets with consistent mass and dosage uniformity. Proper flow is desirable to ensure uniformity in the content and small variation in the final weight of a dosage form, such as, tablets.

Compressibility is required for satisfactory tableting, i.e., the mass must remain in the compact form once the compression force is removed. This means that during the compression process an appropriate compressibility is required in order to obtain stable and intact compact cores of the tablets.

The Carr's Compressibility Index (I) of a material gives a proper indication of the easiness with which this material can be induced to flow (L Lachman et al., 2001). The compressibility index of a material can be calculated by the following equation:

$$\mathbf{I} = [1 - v/v0] \times 100$$

where, v is the volume occupied by a sample of the powder after undergoing a number of standard rate and v0 is the initial volume of the sample before being subjected to this treatment.

The smaller is **I**, the greater is the capacity that the sample has to flow. According to the 7th edition of the European Pharmacopoeia, the tablets must have an **I** between 1% and 25%.

The following table establishes a relation between the Compressibility Index and the flow.

| **I** (%) | Flow |
|---|---|
| 1 - 10 | Excelent |
| 11 - 15 | Good |
| 16 - 20 | Acceptable |
| 21 - 25 | Poor to Acceptable |
| 26 - 31 | Weak |
| 32 - 37 | Very Poor |
| > 38 | Very much weaker |

The Friability Test also allows to evaluate the resistance of the tablets to friction, ensuring that they will remain intact during the coating process, packaging and transport . The test is conducted in an apparatus called friability test equipment, which carries the tablets in free fall repeatedly during a rotating movement, at a speed of 25 rpm. A number of tablets are weighed and placed in the apparatus, where they are exposed to shocks, as they fall 6 cm tall, each time, inside the apparatus. After four minutes of such treatment or 100 rotations, the tablets are weighed and the weight is compared with the initial one. The abrasion loss is a measure of the friability of the tablet. The value is expressed as a percentage. A maximum weight loss of no more than 1 % of the weight of the tablets being tested during friability testing is considered generally acceptable.

Further, the "Visual Appearance of the Tablets " also allows to conclude about the appropriate compressibility by observing the surfaces of tablets , including the lateral surfaces, when they are regular and smooth, showing no signs of abrasion or breakage. This parameter is usually complemented with the observation of punches after the ejection of the tablets, and if there was an appropriate compressibility, the tablets should not show any adherence of powders to

their surfaces.

In the present invention, the said technical advantages are achieved by selecting appropriate and specific excipients to be incorporated in an intimate admixture with the formulation of active ingredients of the dosage forms according to the present invention.

Surprisingly, it was found that the object of the present invention was achieved by incorporating in an intimate admixture medium chain triglycerides or totally or partially hydrogenated oil in the formulation of the solid pharmaceutical composition for oral administration of the present invention.

According to the present invention, the term "Hydrogenated oil" includes either of a partially hydrogenated oil or fully hydrogenated oil. The hydrogenated oils according to the present invention are vegetable oils. Oils (fully) hydrogenated according to the present invention are selected from the group consisting of hydrogenated castor oil, hydrogenated cottonseed oil, hydrogenated soybean oil or mixtures thereof. Partially hydrogenated oils are selected from the group consisting of: partially hydrogenated castor oil, partially hydrogenated cottonseed oil, partially hydrogenated soybean oil or mixtures thereof.

A vegetable oil is a triglyceride extracted from a plant. Unsaturated vegetable oils can be transformed through partial or complete "hydrogenation" into oils of higher melting point. The hydrogenation process involves "sparging" the oil at high temperature and pressure with hydrogen in the presence of a catalyst, typically a powdered nickel compound. As each carbon-carbon double-bond is chemically reduced to a single bond, two hydrogen atoms each form single bonds with the two carbon atoms. The elimination of double bonds by adding hydrogen atoms is called saturation; as the degree of saturation increases, the oil progresses toward being fully hydrogenated. As the degree of saturation increases, the oil's viscosity and melting point increase.

"Medium-chain triglycerides (MCT or MCTs)" according to the present invention refer to mixed triacylglycerols of saturated fatty acids with a chain length of 6-12 carbons, i.e., hexanoic acid (C6:0, common name capronic acid), octanoic acid (C8:0, common name caprylic acid), and decanoic acid (C10:0, common name capric acid). These MCTs can be obtained by conventional methods from natural sources, such as coconut oil, palm kernel oil and bovine milk. Those MCTs obtained from coconut oil or palm kernel oil are produced by hydrolysis of the coconut or palm kernel oil, filtration of MCFAs, and subsequent re-esterification.

The medium chain triglycerides of the present invention are represented by the following formula:

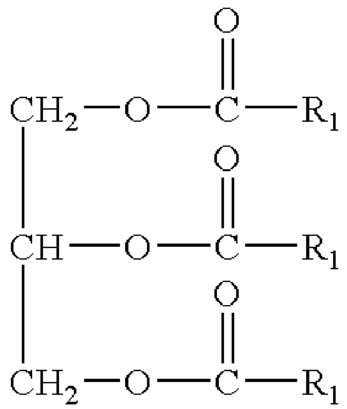

wherein, $R_1$ is independently selected from the group consisting of a fatty acid residue esterified to a glycerol backbone having 6-10 carbons in the carbon backbone ($C_6$ to $C_{12}$ fatty acids). Preferred MCT according to the present invention are commercially available products, such as: Labrafac® Lipophile WL1349 from Gattefosse and Myritol® 318 PH from Cognis.

According to the present invention, the amount of medium chain triglycerides or the oil (total or partially) hydrogenated in the compositions is between 0.001 and 0.25 parts (in weight) to one part (in weight) of ibandronic acid or a pharmaceutically acceptable salt thereof (ibandronate). Preferably, the amount of medium chain triglycerides or totally or partially hydrogenated oil is between 0.01 and 0.08 parts (in weight) to one part (in weight) of ibandronic acid or a pharmaceutically acceptable salt thereof, such as ibandronate. More preferably, the amount of medium chain triglycerides or totally or partially hydrogenated oil is between 0.04 and 0.05 parts (in weight) to one part (in weight) of ibandronic acid or a pharmaceutically acceptable salt thereof, in particular, sodium ibandronate.

Additionally, the pharmaceutical composition may comprise other excipients, provided they are compatible with the active substances of the composition, including, but not limited to, diluents, binders, disintegrants, surfactants, glidants, lubricants , antioxidants or free radicals captors , coating polymers , opacifiers , plasticizers, etc..

As non-limiting examples of diluents according to the present invention, reference is made to microcrystalline cellulose, anhydrous lactose, lactose monohydrate, lactose dihydrate , mannitol , starch, pregelatinized starch and sucrose.

As non-limiting examples of binders according to the present invention, reference is made, to sodium carboxymethylcellulose, microcrystalline cellulose, hydroxypropyl methylcellulose, hydroxypropylcellulose, povidone, starch paste to the pregelatinized starch and sucrose.

As non-limiting examples of disintegrants according to the present invention, reference is made to sodium carboxymethylcellulose, microcrystalline cellulose, croscarmellose sodium, crospovidone , hydroxypropylcellulose, povidone, poloxamer , starch, sodium lauryl sulfate, sodium, pregelatinized starch and starch sodium glycolate.

As non-limiting examples of surfactants according to the present invention, reference is made to poloxamer and sodium lauryl sulfate.

As non-limiting examples of flow agents according to the present invention, reference is made to calcium silicate, starch, talc, colloidal silicon dioxide and sodium aluminum silicate.

As non-limiting examples of lubricants according to the present invention, reference is made to magnesium stearate, sodium stearyl fumarate , PEG, sodium lauryl sulfate and talc.

As non-limiting examples of antioxidants and free radical scavengers according to the present invention, reference is made to butylhydroxyltolueno, butylhydroxylanisole, citric acid and citrate salts, ascorbate salts and ascorbate , alpha- tocopherol, sodium acetate, sodium sulfite and sodium compounds with organic thiol function.

In a preferred embodiment of the present invention, the solid pharmaceutical composition for oral administration comprises ibandronic acid in the form of ibandronate sodium monohydrate, and the amount of Ibandronic acid present in the said composition is 50-200 mg .

Still referring to the same preferred embodiment, the vitamin D compound is cholecalciferol (vitamin D3), which is present in an amount of 11000-44800 UI.

A preferred embodiment of the present invention contemplates formulating the solid pharmaceutical composition for oral administration for a monthly administration regime.

**EXAMPLE 1**

| Constituents | Batch n° IBC004-171T (%) | Batch n° IBC004-251V (%) | Batch n° IBC004-251X (%) |
|---|---|---|---|
| Sodium Ibandronate.H2O | 32,15 | 30,69 | 30,69 |
| Cholecalciferol | 0,11 | 0,10 | 0,10 |
| Antioxidant | 0,10 | 0,08 | 2,04 |
| Lactose | 28,88 | 12,73 | 12,73 |
| Microcrystalline cellulose | 20,19 | 3,28 | 3,28 |
| Povidone | 2,57 | 0,85 | 0,85 |
| Crospovidone | 13,00 | 10,91 | 10,91 |
| Colloidal anhydrous silica | 1,00 | --- | 0,49 |
| Sodium stearyl fumarate | 2,00 | 0,82 | 0,82 |
| Medium chain triglycerides (Labrafac® Lipophile WL1349) | --- | --- | 1,22 |
| Sucrose | --- | 15,48 | 7,13 |
| Starch | --- | 6,54 | 29,75 |
| Partially hydrogenated soybean oil | --- | 3,05 | --- |
| Gelatin | --- | 15,48 | --- |

(Amounts are given as weight % of the total composition)

The manufacturing method of batches IBC004 - 251V and IBC004 - 251X is equivalent. An initial mixture of sodium ibandronate.H2O with lactose is done followed by a granulation with povidone aqueous solution. The granules obtained are dried and calibrated. To the calibrated granules is added the external phase in which there are the medium

chain triglycerides or the partially hydrogenated oil together with the cholecalciferol and at least one of the following excipients: microcrystalline cellulose, crospovidone, antioxidant, starch, gelatin, sucrose and colloidal anhydrous silica. The final blend with sodium stearyl fumarate is used for the compression.

The manufacturing method of batch IBC004 - 171T includes an initial mixture of sodium ibandronateH20, lactose and microcrystalline cellulose followed by a granulation with the antioxidant, pure cholecalciferol, povidone using absolute ethanol. The granules obtained are dried and calibrated. To the calibrated granules is added the external phase in which there is no medium chain triglycerides nor a partially or (fully) hydrogenated oil; in the external phase are microcrystalline cellulose, crospovidone and colloidal anhydrous silica. The final blend with sodium stearyl fumarate is used for the compression.

During the process of compression of batch IBC004 - 171T, it was found that it was not possible to obtain tablets with appropriate characteristics for coating. The tablets showed several signals of abrasion in different surfaces including the lateral surfaces. Simultaneously, there were problems in ejection of the tablets from matrix and in the adherence to the surfaces of the punches. This batch also presented problems of flow.

During compression of batches IBC004 - 251V and IBC004 - 251X, surprisingly tablets became out with smooth surfaces including the lateral surfaces, and there was no trouble in ejection of the tablets from the matrix neither in the adherence to the surfaces of the punches. Additionally these two batches showed no problems as far as flow is concerned.

**EXAMPLE 2** - **Comparative Examples**

The parameters which were evaluated were "Friability", "Compressibility Index" (Carr's Index) and "Visual Appearance of the Tablets".

For this study were submitted compositions according to the invention containing medium chain triglycerides and an oil (total or partially) hydrogenated, the composition described in Example 2 of patent application WO2008074144A1 (in which the alendronate was replaced by ibandronate) and compositions according to the invention in which medium chain triglycerides, or hydrogenated oil has been replaced by a common lubricant (eg calcium stearate, zinc stearate, talc, poloxamer, PEG, sodium lauryl sulfate, myristic acid, palmitic acid or stearic acid).

| Constituents (mg) | IBC005-280A | IBCO05-280B | IBCO05-280C |
|---|---|---|---|
| Ibandronic acid (as sodium Ibandronate) | 160,34 | 160,34 | 160,34 |
| DL-alpha-tocopherol | 2,24 | 2,24 | 2,24 |
| Crystalline sodium ascorbate | 8,96 | 8,96 | 8,96 |
| Vitamin D3 | 0,56 | 0,56 | 0,56 |
| Medium Chain Triglycerides (Myritol® 318PH) | 6,72 | ... . | ... . |
| Sucrose | 39,2 | 39,2 | 39,2 |
| Modified corn starch | 163,63 | 163,63 | 163,63 |
| Silicon dioxide | 2,69 | 2,69 | 2,69 |
| Hydrogenated soybean oil | . . . . | 6,72 | . . . . |
| Mixture of partially hydrogenated cottonseed oil and partially hydrogenated soybean oil | . . . . | . . . . | 6,72 |
| Lactose monohydrate | 70 | 70 | 70 |
| Povidone K30 | 10,1 | 10,1 | 10,1 |
| Lactose monohydrate | 199,26 | 199,26 | 199,26 |
| Crospovidone | 80,3 | 80,3 | 80,3 |
| Sodium stearyl fumarate | 6 | 6 | 6 |
| TOTAL (mg) | 750 | 750 | 750 |

| Example 2 of patent application WO2008074144A1 (in which alendronate was replaced by ibandronate) | mg |
|---|---|
| Ibandronic acid (as sodium ibandronate) | 91,37 |

(continued)

| Example 2 of patent application WO2008074144A1 (in which alendronate was replaced by ibandronate) | mg |
|---|---|
| Dry "sprayed dried" lactose | 98,38 |
| Plasdone (Povidone) | 12,80 |
| Croscarmellose sodium | 13,20 |
| Microcrystalline celulose | 74,90 |
| Vitamina D3 | 26,00 |
| Dioxide colloidal silica | 1,60 |
| Magnesium stearate | 1,75 |
| TOTAL (mg) | 320,00 |

| Constituents (mg) | IBCO06-289A | IBCO06-289B | IBCO06-289C | IBCO06-289D | IBCO06-289E | IBCO06-289F | IBCO06-289G | IBCO06-289H | IBCO06-289I |
|---|---|---|---|---|---|---|---|---|---|
| Ibandronic acid (sodium ibandronate) | 160,34 | 160,34 | 160,34 | 160,34 | 160,34 | 160,34 | 160,34 | 160,34 | 160,34 |
| DL-alpha-tocopherol | 2,24 | 2,24 | 2,24 | 2,24 | 2,24 | 2,24 | 2,24 | 2,24 | 2,24 |
| Crystalline sodium ascorbate | 8,96 | 8,96 | 8,96 | 8,96 | 8,96 | 8,96 | 8,96 | 8,96 | 8,96 |
| Vitamin D3 | 0,56 | 0,56 | 0,56 | 0,56 | 0,56 | 0,56 | 0,56 | 0,56 | 0,56 |
| Sucrose | 39,2 | 39,2 | 39,2 | 39,2 | 39,2 | 39,2 | 39,2 | 39,2 | 39,2 |
| Modified corn starch | 163,63 | 163,63 | 163,63 | 163,63 | 163,63 | 163,63 | 163,63 | 163,63 | 163,63 |
| Silicon dioxide | 2,69 | 2,69 | 2,69 | 2,69 | 2,69 | 2,69 | 2,69 | 2,69 | 2,69 |
| Calcium stearate | 6,72 | | | | | | | | |
| Zinc Stearate | | 6,72 | | | | | | | |
| Talc | | | 6,72 | | | | | | |
| Poloxamer | | | | 7,5 | | | | | |
| PEG | | | | | 7,5 | | | | |
| Sodium lauryl sulfate | | | | | | 7,5 | | | |
| Myristic acid | | | | | | | 7,5 | | |
| Palmitic acid | | | | | | | | 7,5 | |
| Stearic acid | | | | | | | | | 7,5 |
| Lactose monohydrate | 70 | 70 | 70 | 70 | 70 | 70 | 70 | 70 | 70 |
| Povidone K30 | 10,1 | 10,1 | 10,1 | 10,1 | 10,1 | 10,1 | 10,1 | 10,1 | 10,1 |

(continued)

| Constituents (mg) | IBCO06-289A | IBCO06-289B | IBCO06-289C | IBCO06-289D | IBCO06-289E | IBCO06-289F | IBCO06-289G | IBCO06-289H | IBCO06-289I |
|---|---|---|---|---|---|---|---|---|---|
| Lactose monohydrate | 199,26 | 199,26 | 199,26 | 199,26 | 199,26 | 199,26 | 199,26 | 199,26 | 199,26 |
| Crospovidone | 80,3 | 80,3 | 80,3 | 80,3 | 80,3 | 80,3 | 80,3 | 80,3 | 80,3 |
| Sodium stearyl fumarate | 6 | 6 | 6 | 6 | 6 | 6 | 6 | 6 | 6 |
| TOTAL (mg) | 750 | 750 | 750 | 750,78 | 750,78 | 750,78 | 750,78 | 750,78 | 750,78 |

Samples of powders and /or groups of powders are taken for evaluation of their Compressibility Index before compression. Tablet samples are also taken for evaluating the Friability. Friability is measured using the equipment ERWEKA TAR series. To measure the compressibility index is used the equipment ERWEKA SVM series.

| **Batches** | IBCO05-280A | IBCO05-280B | IBCO05-280C |
|---|---|---|---|
| Carr's Compressibility Index (%) | 10 | 11 | 11 |
| Friability | 0,09% | 0,4% | 0,4% |
| Visual appearance of the tablets | smooth surfaces without powder's adherence to the punches | smooth surfaces without powder's adherence to the punches | smooth surfaces without powder's adherence to the punches |

| **Batches** | Example 2 of the patent application WO2008074144A1 |
|---|---|
| Carr's compressibility index (%) | 25 |
| Friability | 0,7% |
| Visual appearance of the tablets | Irregular surfaces and adherence of powders to the punches |

| **Batches** | IBCO06-289A | IBCO06-289B | IBCO06-289C | IBCO06-289D | IBCO06-289E | IBCO06-289F | IBCO06-289G | IBCO06-289H | IBCO06-289I |
|---|---|---|---|---|---|---|---|---|---|
| Carr' s compressibility index (%) | 33 | 35 | 35 | 29 | 33 | 29 | 24 | 28 | 30 |
| Friability | 0,95% | 1.1% | 0,88 % | 0,74 % | 1,07 % | 1,2% | 0,99 % | 1,1% | 0,84 % |
| Visual appearance of the tablets | Irregular surfaces and adherence of powders to punches | Irregular surf aces and adherence of powders to punches | Irregular surfaces and adherenc e of powders to punches | Irregular surf aces and adherence of powders to punches | Irre gular surfaces and adherence of powders to punches | Irre gular surfaces and adherence of powders to punches | Irre gular surfaces and adherenc e of powders to punches | Irregular surfaces and adherence of powders to punches | Irre gular surfaces and adherence of powders to punches |

The present inventors have surprisingly found that, in addition to obtaining tablets with smooth surfaces without adherence of powders to the punches, the compositions comprising a hydrogenated oil or medium chain triglycerides have improved friability, flow and compressibility properties:

* superior compared to the compositions described in patent application WO2008074144A1 which comprise magnesium stearate instead of a hydrogenated oil or a medium chain triglyceride and,

* superior compared to compositions comprising basic lubricants instead of a hydrogenated oil or a medium chain triglyceride.

Obtaining excellent results in terms of physical properties (friability, good compressibility and good flow) and lack of friability and breakage on the surface of the tablets prove the advantages of the present invention.

## Claims

**1.** A solid pharmaceutical composition for oral administration for use in the treatment of bone diseases comprising ibandronic acid or a pharmaceutically acceptable salt, vitamin D, in intimate admixture with medium chain triglycerides or an hydrogenated oil.

**2.** A solid pharmaceutical composition for oral administration for use according to claim 1, wherein ibandronic acid or a pharmaceutically acceptable salt thereof is present in an amount of 50 mg to 200 mg.

**3.** A solid pharmaceutical composition for oral administration for use according to claims 1 and 2, wherein vitamin D is present in an amount of 11000 IU to 44800 IU .

**4.** A solid pharmaceutical composition for oral administration for use according to claims 1 to 3, wherein the hydrogenated oil is a fully hydrogenated oil or a partially hydrogenated oil.

**5.** A solid pharmaceutical composition for oral administration for use according to claims 1 to 4, wherein the hydrogenated oil is a hydrogenated vegetable oil.

**6.** A solid pharmaceutical composition for oral administration for use according to claims 4 to 5, wherein the fully hydrogenated oil is selected from the group consisting of hydrogenated soybean oil, hydrogenated cotton seed oil, hydrogenated castor oil and mixtures thereof.

**7.** A solid pharmaceutical composition for oral administration for use according to claims 4 to 5, wherein the partially hydrogenated oil is selected from the group consisting of partially hydrogenated soybean oil, partially hydrogenated cotton seed oil, partially hydrogenated castor oil and mixtures thereof.

**8.** A solid pharmaceutical composition for oral administration for use according to any of the preceding claims, wherein the amount of medium-chain triglycerides or the oil (total or partially) hydrogenated is between 0.001 to 0.25 parts (in weight) to one part (in weight) of ibandronic acid or a pharmaceutically acceptable salt thereof.

**9.** A solid pharmaceutical composition for oral administration for use according to claim 8, wherein the amount of medium-chain triglycerides or the totally or partially hydrogenated oil is between 0.01 to 0.08 parts (in weight) to one part (in weight) of ibandronic acid or a pharmaceutically acceptable salt thereof.

**10.** A solid pharmaceutical composition for oral administration for use according to claim 9, wherein, the amount of medium chain triglycerides or the totally or partially hydrogenated oils between 0.04 to 0.05 parts (in weight) to one part (in weight) of ibandronic acid or a pharmaceutically acceptable salt thereof.

**11.** A solid pharmaceutical composition for oral administration for use according to any of the preceding claims, comprising additionally at least one of the excipients selected from the group of diluents, binders, disintegrants, surfactants, flow agents, lubricants, antioxidants or free radical captors.

**12.** A solid pharmaceutical composition for oral administration for use according to claim 11, wherein the antioxidants and free radicals captors are selected from the group consisting of butylhydroxyltoluene, butylhydroxylanisole, citric

acid and citrate salts, salts of ascorbic acid or ascorbate , alpha- tocopherol, sodium acetate, sodium sulfite and organic compounds with thiol function.

**13.** A solid pharmaceutical composition for oral administration for use according to claim 12, wherein the antioxidant is selected from the group consisting of ascorbic acid, ascorbate salts or alpha - tocopherol.

**14.** A solid pharmaceutical composition for oral administration for use according to any of the preceding claims, wherein Vitamin D is present in the form of cholecalciferol.

**15.** A solid pharmaceutical composition for oral administration for use according to any of the preceding claims in the form of tablets, capsules or sachets.

**16.** A solid pharmaceutical composition for oral administration for use according to claim 15, in the form of coated tablets.

**17.** A solid pharmaceutical composition for oral administration for use according to any of the preceding claims, formulated for a monthly administration.

**18.** Process for obtaining a solid pharmaceutical composition for oral administration comprising ibandronic acid or a pharmaceutically acceptable salt, vitamin D, in intimate admixture with medium chain triglycerides or an hydrogenated oil comprising:

- an initial mixture of ibandronic acid or a pharmaceutically acceptable salt thereof with an appropriate amount of excipients using an appropriate and conventional mixer
- wet granulation of the previous mixture using a granulation equipment (a fluid bed dryer)
- drying of granules
- calibration of the dried granules using an appropriate and conventional sieve
- addition of the excipients presented in the external phase, where the medium chain triglyceride or, a totally or partially hydrogenated oil is included to improve the properties of flow, compressibility and ejection of the composition
- mix the dried granules with the external phase using a proper mixer
- by means of a tablet press machine, compacting the previous mixture into cores or alternatively, using a filling machine for the obtention of capsules or sachets.

**19.** A process for obtaining a solid pharmaceutical composition for oral administration according to claim 18 wherein the tablets are subsequently coated.

**Patentansprüche**

**1.** Eine feste pharmazeutische Zusammensetzung zur oralen Verabreichung zur Anwendung bei der Behandlung von Knochenerkrankungen umfassend Ibandronsäure oder ein pharmazeutisch geeignetes Salz, Vitamin D, in inniger Beimischung mit mittelkettiger Triglyceride oder hydriertem Öl.

**2.** Eine feste pharmazeutische Zusammensetzung zur oralen Verabreichung zur Anwendung gemäß Anspruch 1, wobei Ibandronsäure oder ein pharmazeutisch geeignetes Salz davon in einer Menge von 50 mg bis 200 mg vorhanden ist.

**3.** Eine feste pharmazeutische Zusammensetzung zur oralen Verabreichung zur Anwendung gemäß Ansprüche 1 und 2, wobei Vitamin D in einer Menge von 11000 IE bis 44800 IE vorhanden ist.

**4.** Eine feste pharmazeutische Zusammensetzung zur oralen Verabreichung zur Anwendung gemäß Ansprüche 1 bis 3, wobei das hydrierte Öl ein vollständig hydriertes oder ein teils hydriertes Öl ist.

**5.** Eine feste pharmazeutische Zusammensetzung zur oralen Verabreichung zur Anwendung gemäß Ansprüche 1 bis 4, wobei das hydrierte Öl ein hydriertes Pflanzenöl ist.

**6.** Eine feste pharmazeutische Zusammensetzung zur oralen Verabreichung zur Anwendung gemäß Ansprüche 4 bis 5, wobei das vollständig hydrierte Öl von der Gruppe bestehend aus hydriertes Sojaöl, hydriertes Baumwollsamenöl,

hydriertes Riziniusöl und Mischungen davon ausgewählt ist.

**7.** Eine feste pharmazeutische Zusammensetzung zur oralen Verabreichung zur Anwendung gemäß Ansprüche 4 bis 5, wobei das teils hydrierte Öl von der Gruppe auserwählt ist, bestehend aus teils hydriertes Sojaöl, teils hydriertes Baumwollsamenöl, teils hydriertes Rizinusöl und Mischungen davon.

**8.** Eine feste pharmazeutische Zusammensetzung zur oralen Verabreichung zur Anwendung gemäß einer der vorigen Ansprüche, wobei die Menge von mittelkettiger Triglyceride oder das (vollständig oder teils) hydrierte Öl zwischen 0.001 bis 0.25 Teilen (in Gewicht) zu einem Teil (in Gewicht) von Ibandronsäure oder einem pharmazeutisch geeigneten Salz davon ist.

**9.** Eine feste pharmazeutische Zusammensetzung zur oralen Verabreichung zur Anwendung gemäß Anspruch 8, wobei die Menge von mittelkettiger Triglyceride oder des vollständig oder teils hydrierten Öls zwischen 0.01 bis 0.08 Teilen (in Gewicht) zu einem Teil (in Gewicht) von Ibandronsäure oder einem pharmazeutisch geeigneten Salz davon ist.

**10.** Eine feste pharmazeutische Zusammensetzung zur oralen Verabreichung zur Anwendung gemäß Anspruch 9, wobei die Menge von mittelkettiger Triglyceride oder den vollständig oder teils hydrierten Ölen zwischen 0.04 bis 0.05 Teilen (in Gewicht) zu einem Teil (in Gewicht) von Ibandronsäure oder einem pharmazeutisch geeigneten Salz davon ist.

**11.** Eine feste pharmazeutische Zusammensetzung zur oralen Verabreichung zur Anwendung gemäß einer der vorigen Ansprüche, umfassend zusätzlich mindestens einen Arzneistoffträger auserwählt von der Gruppe aus Verdünnern, Bindemittel, Sprengmittel, Tensiden, Fließmittel, Gleitmittel, Antioxidantien oder freien Radikalfängern.

**12.** Eine feste pharmazeutische Zusammensetzung zur oralen Verabreichung zur Anwendung gemäß Anspruch 11, wobei die Antioxidantien und freien Radikalfängern von der Gruppe bestehend aus Butylhydroxytoluol, Butyhydroxyanisol, Zitronensäure und Salzcitrate, Salze aus Ascorbinsäure oder Ascorbate, alpha-Tocopherol, Natriumacetat, Natriumsulfit und organische Verbindungen mit Thiol-Funktion ausgewählt sind.

**13.** Eine feste pharmazeutische Zusammensetzung zur oralen Verabreichung zur Anwendung gemäß Anspruch 12, wobei das Antioxidans von der Gruppe bestehend aus Ascorbinsäure, Ascorbatsalze oder alpha-Tocopherol ausgewählt sind.

**14.** Eine feste pharmazeutische Zusammensetzung zur oralen Verabreichung zur Anwendung gemäß einer der vorigen Ansprüche, wobei Vitamin D in der Form von Cholecalciferol vorhanden ist.

**15.** Eine feste pharmazeutische Zusammensetzung zur oralen Verabreichung zur Anwendung gemäß einer der vorigen Ansprüche in der Form von Tabletten, Kapseln oder Päckchen.

**16.** Eine feste pharmazeutische Zusammensetzung zur oralen Verabreichung zur Anwendung gemäß Anspruch 15 in der Form von Filmtabletten.

**17.** Eine feste pharmazeutische Zusammensetzung zur oralen Verabreichung zur Anwendung gemäß einer der vorigen Ansprüche, zubereitet für eine Monatsverabreichung.

**18.** Verfahren zur Erhaltung einer festen pharmazeutischen Mischung zur oralen Anwendung umfassend Ibandronsäure oder ein pharmazeutisch geeignetes Salz, Vitamin D, in inniger Beimischung mit mittelkettiger Triglyceride oder einem hydrierten Öl umfassend:

- eine Erstmischung von Ibandronsäure oder einem pharmazeutisch geeigneten Salz davon mit einer geeigneten Menge von Arzneistoffträgern durch Verwendung eines geeigneten und üblichen Mixers
- Nassgranulation der vorigen Mischungen durch Verwendung einer Granulationsausrüstung (Wirbelschichttrockner)
- Trocknen von Granulate
- Kalibrieren der getrockneten Granulate durch Verwendung eines geeigneten und üblichen Siebes
- Zusatz der Arzneistoffträgern aufgewiesen in der externen Phase, wo die mittelkettige Triglyceride oder, ein vollständig oder teils hydriertes Öl ist zur Besserung der Fließeigenschaften eingeschlossen, Verdichtbarkeit

und Mischungsauswurf
- Mischen der getrockneten Granulate mit der externen Phase durch Verwendung eines geeigneten Mixers
- Verdichten der vorigen Mischung mittels einer Tablettenpressmaschine in Kernstücke oder, andernfalls, Verwendung einer füllenden Maschine zur Gewinnung der Kapseln oder Päckchen.

**19.** Verfahren zur Erhaltung einer festen pharmazeutischen Mischung zur oralen Anwendung gemäß Anspruch 18, wobei die Tabletten hintereinander beschichtet sind.

## Revendications

**1.** Composition pharmaceutique solide pour administration orale pour utilisation dans le traitement des maladies des os, comprenant un acide ibandronique ou un sel pharmaceutiquement acceptable, de la vitamine D, dans un mélange intime avec des triglycérides à chaîne moyenne ou d' huile hydrogénée.

**2.** Composition pharmaceutique solide pour administration orale pour utilisation selon la revendication 1, où l'acide ibandronique où un sel pharmaceutiquement acceptable de celui-ci est présent dans une quantité entre 50 mg et 200 mg.

**3.** Composition pharmaceutique solide pour administration orale pour utilisation selon les revendications 1 et 2, où la vitamine D est présente dans une quantité entre 11000 UI et 44800 UI.

**4.** Composition pharmaceutique solide pour administration orale pour utilisation selon les revendications 1 à 3, où l'huile hydrogénée est une huile entièrement hydrogénée ou une huile partiellement hydrogénée.

**5.** Composition pharmaceutique solide pour administration orale pour utilisation selon les revendications 1 à 4, où l'huile hydrogénée est une huile végétale hydrogénée.

**6.** Composition pharmaceutique solide pour administration orale pour utilisation selon les revendications 4 à 5, où l'huile entièrement hydrogénée est sélectionnée d'un groupe consistant en de l'huile de soja hydrogénée, de l'huile de graine de coton hydrogénée, de l'huile de ricin hydrogénée et de mélanges de celles-ci.

**7.** Composition pharmaceutique solide pour administration orale pour utilisation selon les revendications 4 à 5, où l'huile partiellement hydrogénée est sélectionnée du groupe composé d'huile de soja partiellement hydrogénée, d'huile de graine de coton partiellement hydrogénée, d'huile de ricin partiellement hydrogénée et de mélanges de celles-ci.

**8.** Composition pharmaceutique solide pour administration orale pour utilisation selon une quelconque revendication précédente, où la quantité des triglycérides à chaîne moyenne ou de l'huile (totalement ou partiellement) hydrogénée est entre 0,001 et 0,25 parties (en poids) pour une partie (en poids) d'acide ibandronique ou un sel pharmaceutiquement acceptable de celui-ci.

**9.** Composition pharmaceutique solide pour administration orale pour utilisation selon la revendication 8, où la quantité de triglycérides à chaîne moyenne ou l'huile partiellement hydrogénée est entre 0,01 et 0,08 parties (en poids) pour une partie (en poids) d'acide ibandronique ou d'un sel pharmaceutiquement acceptable de celui-ci.

**10.** Composition pharmaceutique solide pour administration orale pour utilisation selon la revendication 9, où la quantité de triglycérides à chaîne moyenne ou les huiles partiellement ou totalement hydrogénées entre 0,04 et 0,05 parties (en poids) pourune partie (en poids) d'acide ibandronique ou d'un sel pharmaceutiquement acceptable de celui-ci.

**11.** Composition pharmaceutique solide pour administration orale, pour utilisation selon une quelconque revendication précédente, comprenant additionnellement au moins l'un des excipients sélectionnés du groupe de diluants, liants, désintégrants, tensioactifs, agents d'écoulement, lubrifiants, antioxydants ou capteurs de radicaux libres.

**12.** Composition pharmaceutique solide pour administration orale pour utilisation selon la revendication 11, où les antioxydants et les capteurs de radicaux libres sont sélectionnés du groupe consistant en du butylhydroxytoluène, du butylhydroxylanisole, de l'acide citrique et des sels de citrate, sels de d'acide ascorbique ou ascorbate, de l'alpha-tocophérol, de l'acétate de sodium, du sulfite de sodium et des composés organiques avec une fonction thiol.

**13.** Composition pharmaceutique solide pour administration orale pour utilisation selon la revendication 12, où l'antioxydant est sélectionné du groupe consistant en de l'acide ascorbique, des sels d'ascorbate ou de l'alpha-tocophérol.

**14.** Composition pharmaceutique solide pour administration orale pour utilisation selon une quelconque revendication précédente, où la Vitamine D est présente sous la forme de cholécalciférol.

**15.** Composition pharmaceutique solide pour administration orale pour utilisation selon une quelconque revendication précédente, sous la forme de comprimés, de gélules ou de sachets.

**16.** Composition pharmaceutique solide pour administration orale pour utilisation selon la revendication 15, sous la forme de comprimés enrobés.

**17.** Composition pharmaceutique solide pour administration orale pour utilisation selon une quelconque revendication précédente, formulée pour une administration mensuelle.

**18.** Processus d'obtention d'une composition pharmaceutique solide pour administration orale comprenant de l'acide ibandronique ou un sel pharmaceutiquement acceptable, de la vitamine D, dans une mélange intime avec des triglycérides à chaîne moyenne ou une huile hydrogénée, comprenant :

- mélange initial d'acide ibandronique ou un sel pharmaceutiquement acceptable de celui-ci avec une quantité appropriée d'excipients en utilisant un Mélangeur approprié et conventionnel
- granulation humide du mélange précédent en utilisant un équipement de granulation (séchoir à lit fluidisé)
- séchage de granules
- calibrage des granulés séchés en utilisant un tamis approprié et conventionnel
- addition des excipients présentés dans la phase externe, où le triglycéride à chaîne moyenne ou, une huile totalement ou partiellement hydrogénée est inclus(e) afin d'améliorer les propriétés d'écoulement, de compressibilité et d'éjection de la composition
- mélanger les granulés secs avec la phase externe en utilisant un mélangeur approprié
- moyennant une machine de pressage de tablettes, en compactant le mélange précédent en noyaux ou alternativement, en utilisant une machine de remplissage pour l'obtention de capsules ou de sachets.

**19.** Processus d'obtention d'une composition pharmaceutique solide pour administration orale selon la revendication 18 où les comprimés sont subséquemment enrobés.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2001028564 A1
- WO 2003086415 A1
- WO 2008116809 A1
- WO 2008074144 A1


### Non-patent literature cited in the description


- Development of biphosphonates. *Breast Cancer Res,* 2002, vol. 4, 30-34
- **M.KIM.** *The safety, tolerability and serum ibandronate pharmacokinetic profile of DP-R206, fixed dose ibandronate and cholecalciferol, combination compared with ibandronate 150mg tablet,* 01 February 2013, s70-s71